# EUROPÄISCHE PATENTSCHRIFT

(11) **EP 2 025 357 B1**
(45) Veröffentlichungstag und Bekanntmachung des Hinweises auf die Patenterteilung: **21.05.2014**
(21) Anmeldenummer: 08013511.4
(22) Anmeldetag: 26.07.2008
(51) Int. Cl.: B23K 26/00, B23K 26/073, A61M 5/34

(54) **Spritzenkörper und Verfahren zur Herstellung eines Spritzenkörpers**
Syringe body and method for manufacturing a syringe body
Corps de seringue et procédé de fabrication d'un tel corps de seringue

(30) Priorität: 09.08.2007 DE 102007037565
(43) Veröffentlichungstag der Anmeldung: 18.02.2009
(73) Patentinhaber: Nipro Glass Germany AG, 97702 Münnerstadt (DE)
(72) Erfinder: Heidl, Wolfgang, 97702 Münnerstadt (DE); Gebauer, Hendrik, 04509 Delitzsch (DE); Richter, Lars, 04849 Kossa (DE)
(74) Vertreter: Blind, Julia

(56) Entgegenhaltungen:
- WO-A-2006/136364
- CH-A- 457 727
- GB-A- 981 378
- JP-A- 2000 167 049
- US-A1- 2002 138 042

## Beschreibung

Die Erfindung betrifft einen Spritzenkörper, der einen zumindest teilweise zylindrischen Abschnitt aufweist, wobei in einem axialen Endbereich des zylindrischen Abschnitts eine Kanüle befestigt ist, wobei die Kanüle in einem Aufnahmekanal im axialen Endbereich des zylindrischen Abschnitts angeordnet ist und wobei die Festlegung der Kanüle im Aufnahmekanal an mindestens einem Befestigungsbereich durch Material des zylindrischen Abschnitts erfolgt, das nach einem Aufschmelzen sich um den Umfang der Kanüle dichtend anlegt und eine Verbindung zwischen zylindrischem Abschnitt und Kanüle bildet. Des weiteren betrifft die Erfindung ein Verfahren zur Herstellung eines Spritzenkörpers.

Spritzenkörper und Verfahren zu deren Herstellung sind im Stand der Technik hinlänglich bekannt. In der DE 101 08 958 A1 ist ein Verfahren beschrieben, mit dem eine Kanüle in einem aus Glas bestehenden zylindrischen Abschnitt des Spritzenkörpers und namentlich in dessen axialen Endbereich dadurch festgelegt wird, dass Glasmaterial im axialen Endbereich mittels eines Lasers aufgeschmolzen wird. Die Glasschmelze legt sich um den Umfang der Kanüle an. Beim Abkühlen (Erstarren) des aufgeschmolzenen Glases entsteht eine feste Verbindung zwischen dem zylindrischen Abschnitt des Spritzenkörpers und der Kanüle.

Der Vorteil dieses Verfahrens besteht darin, dass auf den Einsatz eines Klebers zur Festlegung der Kanüle im zylindrischen Körper verzichtet werden kann. Der Einsatz von Klebern ist vor allem unter dem Gesichtspunkt bedenklich, dass hierdurch das zu injizierende Medikament beeinflusst werden kann.

Lösungen, bei denen zwischen der Kanüle und dem zylindrischen Abschnitt eine dauerhafte Verbindung durch Kleben oder durch das Aufschmelzen von Material des zylindrischen Abschnitts hergestellt wird, sind auch aus der CH 457 727 A**,** aus der JP 2000 167049 A**,** aus der GB 981 378 A**,** aus der US 2002/138042 A1 und aus der WO 2006/136364 A bekannt.

Nachteilig ist indes, dass unter ungünstigen Fertigungsumständen die Festigkeit der hergestellten Verbindung zwischen zylindrischem Abschnitt des Spritzenkörpers und Kanüle nicht immer ausreichend ist. Dies gilt insbesondere, wenn ein Biegemoment durch die Verbindung aufgenommen werden soll, das senkrecht auf der Längsachse der Kanüle steht. In diesem Falle muss der in axialer Richtung relativ kurze Befestigungsbereich das Moment abstützen.

Ein anderes Problem besteht darin, dass konstruktionsbedingt in dem axialen Endbereich der Kanüle, der dem Innenraum des zylindrischen Abschnitts zugewandt ist und in dem das zu injizierende Medikament vor der Injektion aufgenommen wird, ein gewisses Totraumvolumen besteht. Dieses Volumen kann bei der Injektion nicht ausgespritzt werden. Dies ist insbesondere bei Medikamenten nachteilig, die sehr kostspielig sind.

Schließlich wird in nachteiliger Weise gelegentlich, wenn auch selten beobachtet, dass es zu einem Herausfallen der Kanüle aus dem Spritzenkörper kommen kann, was auf Probleme während des Einschmelzens der Kanüle in das Glasmaterial schließen lässt.

Der Erfindung liegt daher die **Aufgabe** zugrunde, einen Spritzenkörper der eingangs genannten Art sowie ein zugehöriges Verfahren zu seiner Herstellung so weiterzuentwickeln, bei dem die genanten Nachteile behoben oder zumindest reduziert werden. Demnach wird angestrebt, dass sich eine verbesserte Abstützung der Kanüle im axialen Endbereich des zylindrischen Abschnitts des Spritzenkörpers ergibt, so dass Biegemomente verbessert aufgenommen werden können. Ferner wird angestrebt, das Totraumvolumen zu reduzieren, so dass von dem im zylindrischen Abschnitt aufgenommenen Medikament ein höherer Anteil injiziert werden kann. Die Möglichkeit der Sterilisation des Spritzenkörpers soll ebenfalls verbessert werden. Angestrebt wird schließlich auch, dass die Kanüle im zylindrischen Abschnitt des Spritzenkörpers einen verbesserten Halt hat, so dass die Gefahr eines Ausfallens der Kanüle reduziert wird.

Die **Lösung** dieser Aufgabe durch die Erfindung ist dadurch gekennzeichnet, dass mindestens zwei im axialen Abstand angeordnete Befestigungsbereiche vorhanden sind, die die Kanüle im axialen Endbereich des zylindrischen Abschnitts festlegen, wobei zwischen den beiden Befestigungsbereichen ein Bereich angeordnet ist, in dem keine Verbindung durch aufgeschmolzenes Material des zylindrischen Abschnitts zwischen zylindrischem Abschnitt und Kanüle vorliegt.

Bevorzugt sind zwei Befestigungsbereiche vorhanden, von denen einer am Ende des axialen Endbereichs des zylindrischen Abschnitts angeordnet ist.

Der am Ende des axialen Endbereichs des zylindrischen Abschnitts angeordnete Befestigungsbereich kann am äußeren Umfang eine Kegelfläche oder eine konvexe Fläche bilden.

Es können weiterhin zwei Befestigungsbereiche vorhanden sein, von denen einer an dem axialen Ende der Kanüle angeordnet ist, das dem Inneren des zylindrischen Abschnitts zugewandt ist. In diesem Falle hat es sich bewährt, wenn sich der Innenraum des Spritzenkörpers vom Befestigungsbereich am axialen Ende der Kanüle aus kegelförmig erweitert, wobei die Oberfläche des Befestigungsbereichs eine ebene Form aufweist. Alternativ kann die Oberfläche des Befestigungsbereichs eine gewölbte, insbesondere konvexe, Form aufweisen.

Die Oberfläche des Befestigungsbereichs und das axiale Ende der Kanüle schließen bevorzugt bündig ab. Dies minimiert das Totraumvolumen. Eine alternative Lösung sieht vor, dass das axiale Ende der Kanüle über die Oberfläche des Befestigungsbereichs etwas übersteht und dem Inneren des zylindrischen Abschnitts zugewandt ist.

Der Spritzenkörper besteht dabei bevorzugt, aber nicht zwingend, aus Glas.

Das Verfahren zur Herstellung eines Spritzenkörpers der genannten Art zeichnet sich dadurch aus, dass der Aufschmelz- und Abkühlungsvorgang in mindestens zwei im axialen Abstand angeordneten Befestigungsbereichen des axialen Endbereichs des zylindrischen Abschnitts vorgenommen wird, wobei zwischen den beiden Befestigungsbereichen ein Bereich belassen wird, in dem kein Aufschmelz- und Abkühlungsvorgang erfolgt, um in diesem Bereich keine Verbindung durch aufgeschmolzenes Material des zylindrischen Abschnitts zwischen zylindrischem Abschnitt und Kanüle herzustellen.

Das Aufschmelzen erfolgt dabei bevorzugt durch einen Laser, wobei es sich um einen Nd:YAG-Laser, einen Dioden-Laser oder auch um einen CO₂ Laser handeln kann.

Das Aufschmelzen und Abkühlen des Materials in den mindestens zwei Befestigungsbereichen kann sequentiell, d. h. in zeitlicher Abfolge erfolgen.

Es ist alternativ aber auch möglich, dass das Aufschmelzen und Abkühlen des Materials in den mindestens zwei Befestigungsbereichen gleichzeitig erfolgt.

Das Laserlicht kann mittels einer Fokussierungs-Optik so auf den Befestigungsbereich gelenkt werden, dass sich dort eine kreisringformige Aufheizzone um die Kanüle herum ausbildet.

Alternativ kann vorgesehen werden, dass beim Aufschmelzen des Materials des Befestigungsbereichs der Laserstrahl ortsfest ist und der Spritzenkörper so rotiert, dass eine ringförmige Aufheizzone entsteht. In kinematischer Umkehr kann in diesem Falle natürlich auch vorgesehen werden, dass beim Aufschmelzen der Spritzenkörper ortsfest ist und der Laserstrahl so bewegt wird, dass eine ringförmige Aufheizzone um die Kanüle herum entsteht.

Mit der vorgeschlagenen Lösung wird erreicht, dass eine sehr feste Verbindung zwischen der Kanüle und dem zylindrischen Abschnitt des Spritzenkörpers sichergestellt ist. Die Gefahr eines Herausfallens der Kanüle ist deutlich reduziert, da jetzt mindestens zwei Befestigungsbereiche zusammen den Halt der Kanüle im Spritzenkörper sicherstellen.

Ferner wird mit dem Erfindungsvorschlag eine verbesserte Sterilisation des Spritzenkörpers ermöglicht. Dabei können alle bekannten Sterilisationsverfahren eingesetzt werden.

Durch die bevorzugte Ausgestaltung des axial innenliegenden Befestigungsbereichs kann das Totraumvolumen deutlich reduziert werden, was entsprechende Vorteile bietet, wenn kostspielige Medikamente verabreicht werden müssen.

Schließlich werden auch Biegemomente jetzt sehr viel sicherer aufgenommen, da die beiden (bzw. die mindestens zwei) axial beabstandeten Befestigungsbereiche zu geringen Kräften in den jeweiligen Auflagestellen führen.

In der Zeichnung sind Ausführungsbeispiele der Erfindung dargestellt. Es zeigen:
- Fig. 1: in perspektivischer Ansicht einen Spritzenkörper mit zylindrischem Abschnitt und Kanüle,
- Fig. 2: einen Schnitt durch den axialen Endbereich des zylindrischen Abschnitts des Spritzenkörpers mit befestigter Kanüle,
- Fig. 3: eine perspektivische Ansicht der Anordnung gemäß Fig. 2,
- Fig.4: den axialen Endbereich des zylindrischen Abschnitts des Spritzenkörpers mit befestigter Kanüle gemäß einer alternativen Ausführungsform,
- Fig. 5: eine Schemadarstellung für die Herstellung der Anordnung gemäß Fig. 4 mit Strahlteiler für einen Laserstrahl und
- Fig. 6: eine Fokussierungs-Optik für einen Laserstrahl zur Erzeugung einer kreisringförmigen Aufheizzone.

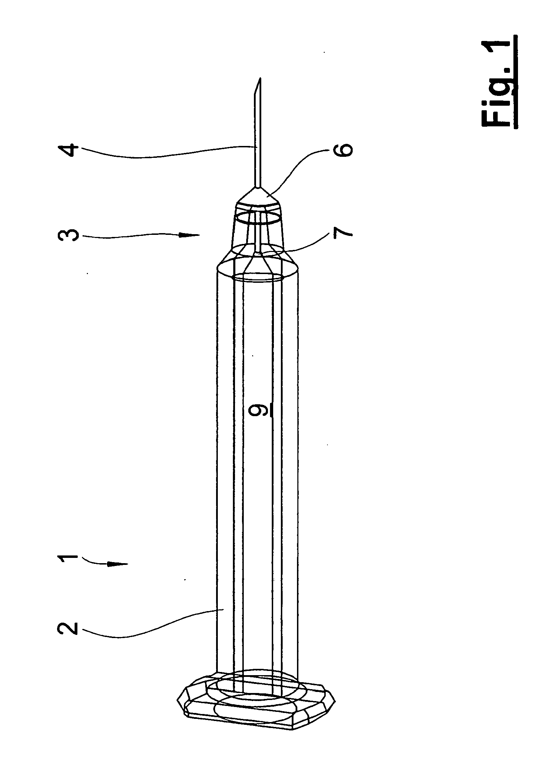

In Fig. 1 ist ein Spritzenkörper 1 zu erkennen, der als Hauptteil einen zylindrischen (bzw. hohlzylindrischen) Abschnitt 2 aufweist, der einen Innenraum 9 hat, in den das zu injizierende Medikament aufgenommen wird. In bekannter Weise wird durch einen nicht dargestellten Kolben und dessen axiale Betätigung das Medikament im Innenraum 9 injiziert. Dies erfolgt in ebenfalls bekannter Weise beispielsweise intravenös durch eine Kanüle 4, die im axialen Endbereich 3 des zylindrischen Abschnitts 2 im Spritzenkörper 1 festgelegt ist.

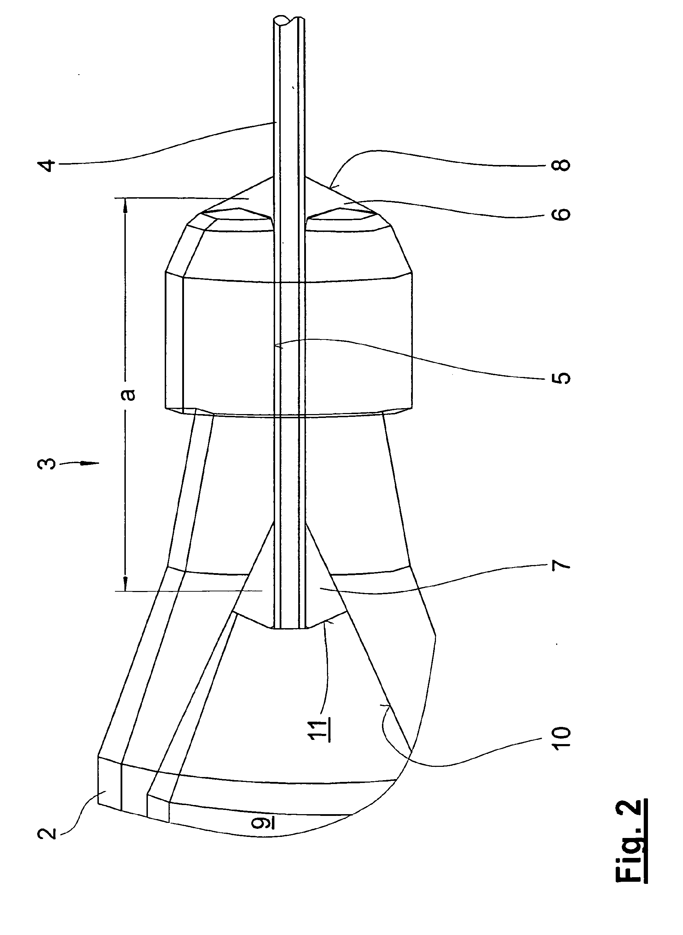

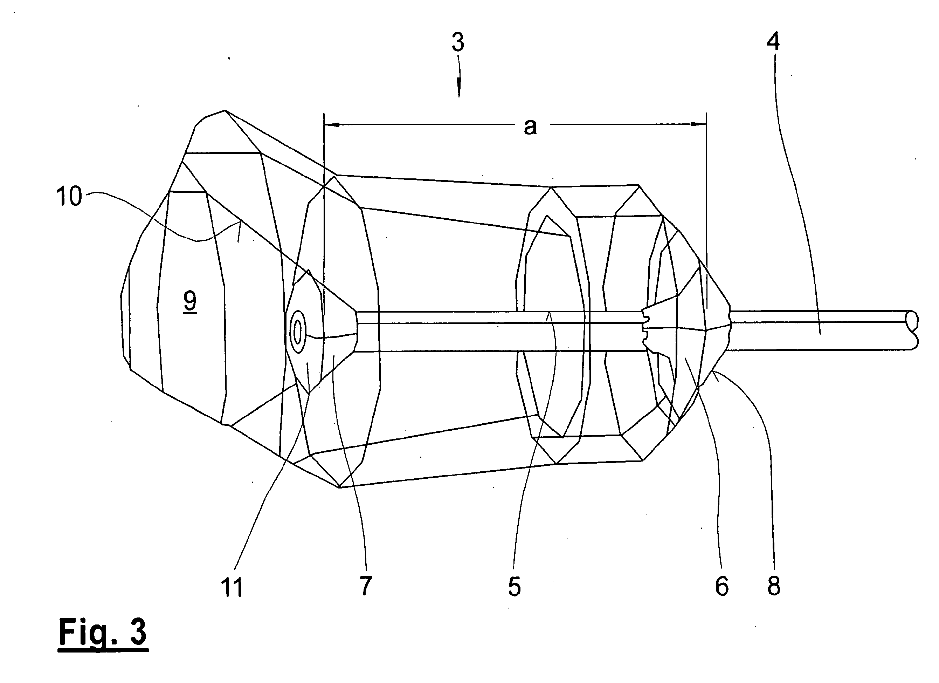

Erfindungsgemäß ist die Kanüle 4 mittels (mindestens) zwei im axialen Abstand a (s. Figuren 2 und 3) angeordneten Befestigungsbereichen 6 und 7 im axialen Endbereich 3 des zylindrischen Abschnitts 2 festgelegt. Der axiale Abstand a beträgt vorzugsweise das 3- bis 60-fache oder zumindest das 3- bis 30-fache des Außendurchmessers der Kanüle 4, besonders bevorzugt das 5-bis 22-fache desselben.

Details einer bevorzugten Ausführungsform der Erfindung sind in den Figuren 2 und 3 zu sehen.

Hier ist zu erkennen, dass in den axialen Endbereich 3 des zylindrischen Abschnitts 2 ein Aufnahmekanal 5 - ausgeführt als zylindrische Bohrung - eingearbeitet ist. In den Aufnahmekanal 5 wird die Kanüle 4 eingeschoben, bis die dargestellte Relativposition zwischen Endbereich 3 und Kanüle 4 gegeben ist.

Dann wird mittels eines Lasers der Befestigungsbereich 6 und 7 aufgeschmolzen, so dass sich die Schmelze des Glases in den Bereichen 6 und 7 um den Umfang der Kanüle 4 legt. Nach dem Abkühlen der Schmelze und deren Erstarren liegt eine feste Verbindung zwischen der Kanüle 4 und dem Spritzenkörper in den beiden Befestigungsbereichen 6 und 7 vor, die als Befestigungspunkte ausgeführt sind.

Das Aufschmelzen und Erstarren erfolgt bei den Ausführungsbeispielen gemäß der Figuren 2 und 3 so, dass sich in dem Befestigungsbereich 6 eine Kegelfläche 8 ausbildet.

Was den anderen Befestigungsbereich 7 anbelangt, liegt dieser in einem sich konisch bzw. kegelig aufweitenden Bereich, in dem der axiale Endbereich 3 in den zylindrischen Abschnitt 2 übergeht. D. h. hier liegt im Inneren 9 des zylindrischen Körpers bzw. des axialen Endbereichs 3 eine Kegelform 10 vor.

Der Befestigungsbereich 7 ist dabei nach dem Schmelzen und Wiedererstarren so ausgebildet, dass sich eine bevorzugt konvexe Oberfläche 11 ergibt, die auf den Innenraum 9 zu gerichtet ist. Wie weiterhin zu sehen ist, können das axiale (linke) Ende der Kanüle 4 und die Oberfläche 11 des Befestigungsbereichs 7 bündig abschließen. Es ist alternativ aber auch möglich, dass die Kanüle 4 etwas aus dem aufgeschmolzenen Glasmaterial heraussteht und dem Inneren 9 des zylindrischen Abschnitts 2 zugewandt ist.

Durch diese Ausgestaltung wird eine Minimierung des Totraumvolumens im Spritzenkörper erreicht, d. h. eine Minimierung des Medikamenten-Volumens, das aus der Spritze nicht injiziert werden kann. Der (nicht dargestellte) Kolben kann entsprechend kongruent zur Oberfläche 11 ausgebildet werden.

Ferner ist sofort ersichtlich, dass aufgrund der Beabstandung der beiden Befestigungsbereiche 6 und 7 um einen axialen Abstand a durch die Kanüle 4 aufgegebene Biegemomente viel besser aufgenommen werden können, als dies beim Stand der Technik mit nur einem Befestigungsbereich möglich ist.

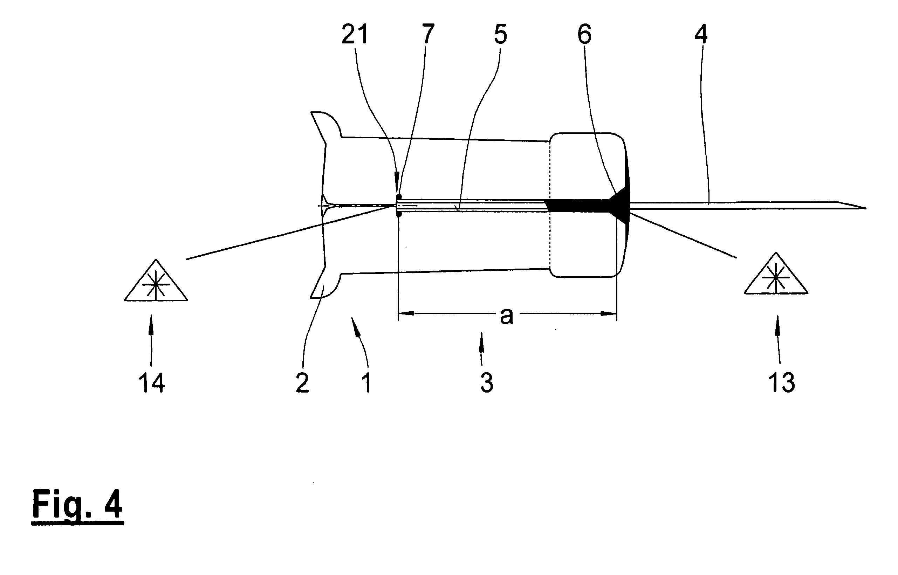

In Fig. 4 ist schematisch dargestellt, wie die Herstellung der beiden Befestigungsbereiche 6 und 7 erfolgt. Hier sind zwei Laser 13 und 14 vorgesehen, von denen jeder einen der beiden Befestigungsbereiche 6 bzw. 7 erhitzt und das Glasmaterial dort aufschmilzt.

Zu der Lösung gemäß Fig. 4 sei noch angemerkt, dass hier insofern eine alternative Ausgestaltung des Spritzenkörpers vorliegt, als hier der Aufnahmekanal 5 nicht durchgängig ausgebildet ist, sondern sich - von außen kommend - über eine gewisse axiale Erstreckung ausdehnt und dann in eine Bohrung mit verkleinertem Durchmesser übergeht, durch den das Medikament eingespritzt wird.

Demgemäß wird die Kanüle 4 hier bis zu einem Anschlag 21 in den axialen Endbereich 3 des zylindrischen Abschnitts 2 des Spritzenkörpers eingeschoben und in der erläuterten Weise mittels der Laser 13 und 14 fixiert.

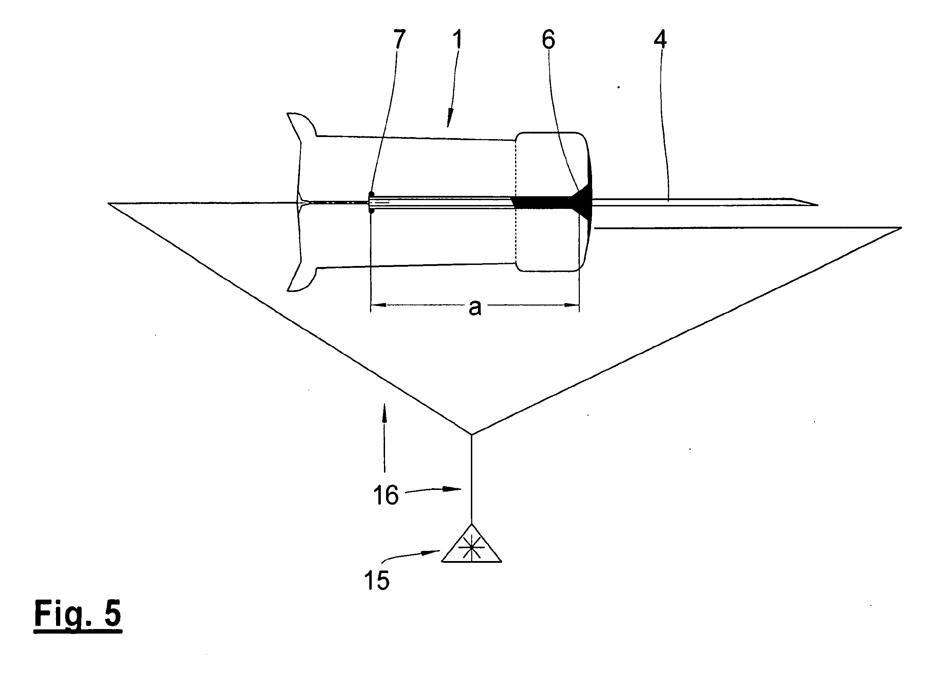

Bei der Lösung gemäß Fig. 5 ist vorgesehen, dass nicht zwei Laser, sondern nur ein einziger Laser 15 zum Einsatz kommt, dessen Licht allerdings über einen nur schematisch dargestellten Strahlteiler 16 aufgeteilt und zu den beiden Stellen geleitet wird, die aufgeschmolzen werden sollen, um die Befestigungsbereiche 6 und 7 herzustellen.

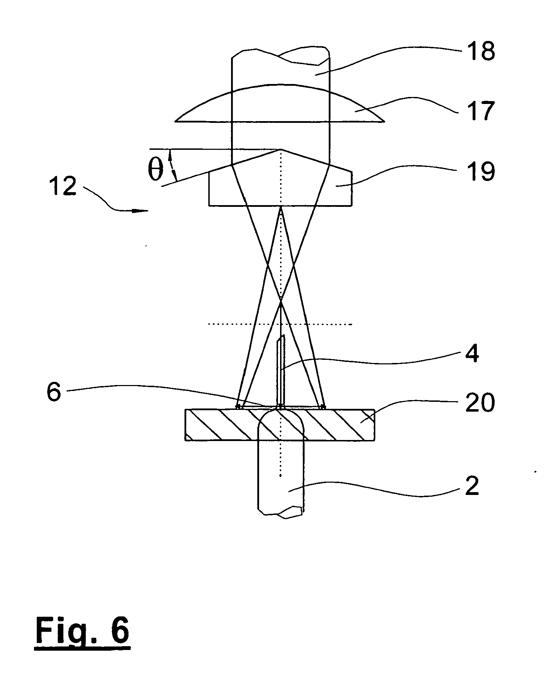

Eine fertigungstechnische bevorzugte Lösung zum Aufschmelzen eines Ringbereichs Glas um die Kanüle 4 herum ist in Fig. 6 zu sehen. Hier ist eine Fokussierungs-Optik 12 gezeigt, in die ein Laserstrahl 18 eintritt und auf eine Fokussierlinse 17 geleitet wird. Mittels eines Prismas (mit Prismenwinkel Θ) wird das Licht gebündelt und auf das Werkstück 20 geleitet, wo sich ein ringförmiger Bereich ausbildet, der mittels des Laserstrahls 18 aufgeschmolzen werden kann.

Diese als "Axicon" bezeichnete Vorrichtung ermöglicht also die Erzeugung einer ringförmigen Aufschmelzung von Glas als Alternative zum Ausführen einer relativen Drehung zwischen Spritzenkörper und Laserstrahl.

Es ist stets nur eine partielle und möglichst begrenzte Aufschmelzung des Glasmaterials angestrebt, um keine Formeinbußen am Spritzenkörper hervorzurufen.

Es können neben CO₂-Lasern auch Festkörperlaser eingesetzt werden. Mit letzteren ist es möglich, das Glas zu durchstrahlen und die Kanüle im Inneren des Glaskörpers bzw. das Glasmaterial um die Kanüle herum zu erhitzen. Ein CO₂-Laserstrahl kann das Glas indes nicht durchdringen.

Die Leistung der zum Einsatz kommenden Laser wird geregelt, so dass die Temperatur der Schweißstelle nach einer starken Erhitzung mit hohem Temperaturgradienten konstant bleibt. Durch eine genaue Führung der Temperatur kann ein hoher Grad an Reproduzierbarkeit im Prozess erreicht werden.

Hinsichtlich Details zu der fraglichen Laser-Technologie wird ausdrücklich auf die DE 198 39 343 A1 Bezug genommen.

### Bezugszeichenliste:

- 1: Spritzenkörper
- 2: zylindrischer Abschnitt
- 3: axialer Endbereich
- 4: Kanüle
- 5: Aufnahmekanal
- 6: Befestigungsbereich
- 7: Befestigungsbereich
- 8: Kegelfläche
- 9: Inneres des zylindrischen Abschnitts / Innenraum
- 10: Kegelform
- 11: Oberfläche des Befestigungsbereichs
- 12: Fokussierungs-Optik
- 13: erster Laser
- 14: zweiter Laser
- 15: Laser
- 16: Strahlteiler
- 17: Fokussierlinse
- 18: Laserstrahl
- 19: Prisma
- 20: Werkstück
- 21: Anschlag

- a: axialer Abstand
- Θ: Winkel

## Patentansprüche

1. Spritzenkörper (1), der einen zumindest teilweise zylindrischen Abschnitt (2) aufweist, wobei in einem axialen Endbereich (3) des zylindrischen Abschnitts (2) eine Kanüle (4) befestigt ist, wobei die Kanüle (4) in einem Aufnahmekanal (5) im axialen Endbereich (3) des zylindrischen Abschnitts (2) angeordnet ist und wobei die Festlegung der Kanüle (4) im Aufnahmekanal (5) an mindestens einem Befestigungsbereich (6, 7) durch Material des zylindrischen Abschnitts (2) erfolgt, das nach einem Aufschmelzen sich um den Umfang der Kanüle (4) dichtend anlegt und eine Verbindung zwischen zylindrischem Abschnitt (2) und Kanüle (4) bildet,
**dadurch gekennzeichnet,**
**dass** mindestens zwei im axialen Abstand (a) angeordnete Befestigungsbereiche (6, 7) vorhanden sind, die die Kanüle (4) im axialen Endbereich (3) des zylindrischen Abschnitts (2) festlegen, wobei zwischen den beiden Befestigungsbereichen (6, 7) ein Bereich angeordnet ist, in dem keine Verbindung durch aufgeschmolzenes Material des zylindrischen Abschnitts (2) zwischen zylindrischem Abschnitt (2) und Kanüle (4) vorliegt.

2. Spritzenkörper nach Anspruch 1, **dadurch gekennzeichnet, dass** zwei Befestigungsbereiche (6, 7) vorhanden sind, von denen einer am Ende des axialen Endbereichs (3) des zylindrischen Abschnitts (2) angeordnet ist.

3. Spritzenkörper nach Anspruch 2, **dadurch gekennzeichnet, dass** der am Ende des axialen Endbereichs (3) des zylindrischen Abschnitts (2) angeordnete Befestigungsbereich (6) am äußeren Umfang eine Kegelfläche (8) oder eine konvexe Fläche bildet.

4. Spritzenkörper nach einem der Ansprüche 1 bis 3, **dadurch gekennzeichnet, dass** zwei Befestigungsbereiche (6, 7) vorhanden sind, von denen einer an dem axialen Ende der Kanüle (4) angeordnet ist, das dem Inneren (9) des zylindrischen Abschnitts (2) zugewandt ist.

5. Spritzenkörper nach Anspruch 4, **dadurch gekennzeichnet, dass** sich der Innenraum (9) des Spritzenkörpers (1) vom Befestigungsbereich (7) am axialen Ende der Kanüle (4) aus kegelförmig (10) erweitert, wobei die Oberfläche (11) des Befestigungsbereichs (7) eine ebene oder eine gewölbte, insbesondere konvexe, Form aufweist.

6. Spritzenkörper nach Anspruch 5, **dadurch gekennzeichnet, dass** die Oberfläche (11) des Befestigungsbereichs (7) und das axiale Ende der Kanüle (4) bündig abschließen.

7. Spritzenkörper nach einem der Ansprüche 1 bis 6, **dadurch gekennzeichnet, dass** der zylindrische Abschnitt (2) aus Glas besteht.

8. Verfahren zur Herstellung eines Spritzenkörpers (1), bei dem in einen Aufnahmekanal (5) im axialen Endbereich (3) eines zumindest teilweise zylindrischen Abschnitts (2) des Spritzenkörpers (1) eine Kanüle (4) eingesetzt und dann in mindestens einem Befestigungsbereich (6, 7) Material des axialen Endbereichs (3) des zylindrischen Abschnitts (2) durch eine Wärmequelle aufgeschmolzen wird, dass dann das Material abkühlen gelassen wird, so dass es sich dichtend um die Kanüle (4) anlegt und eine Verbindung zwischen dem axialen Endbereich (3) des zylindrischen Abschnitts (2) und der Kanüle (4) bildet,
**dadurch gekennzeichnet,**
**dass** der Aufschmelz- und Abkühlungsvorgang in mindestens zwei im axialen Abstand (a) angeordneten Befestigungsbereichen (6, 7) des axialen Endbereichs (3) des zylindrischen Abschnitts (2) vorgenommen wird, wobei zwischen den beiden Befestigungsbereichen (6, 7) ein Bereich belassen wird, in dem kein Aufschmelz- und Abkühlungsvorgang erfolgt, um in diesem Bereich keine Verbindung durch aufgeschmolzenes Material des zylindrischen Abschnitts (2) zwischen zylindrischem Abschnitt (2) und Kanüle (4) herzustellen.

9. Verfahren nach Anspruch 8, **dadurch gekennzeichnet, dass** die Wärmequelle eine elektromagnetische Strahlung emittiert.

10. Verfahren nach Anspruch 8 oder 9, **dadurch gekennzeichnet, dass** das Aufschmelzen durch einen Laser, insbesondere durch einen Nd:YAG-Laser oder einen Dioden-Laser oder einen CO₂- Laser, erfolgt.

11. Verfahren nach einem der Ansprüche 8 bis 10, **dadurch gekennzeichnet, dass** das Aufschmelzen und Abkühlen des Materials in den mindestens zwei Befestigungsbereichen (6, 7) sequentiell erfolgt.

12. Verfahren nach einem der Ansprüche 8 bis 10, **dadurch gekennzeichnet, dass** das Aufschmelzen und Abkühlen des Materials in den mindestens zwei Befestigungsbereichen (6, 7) gleichzeitig erfolgt.

13. Verfahren nach einem der Ansprüche 10 bis 12, **dadurch gekennzeichnet, dass** das Laserlicht mittels einer Fokussierungs-Optik (12) so auf den Befestigungsbereich (6, 7) gelenkt wird, dass sich dort eine kreisringförmige Aufheizzone ausbildet.

14. Verfahren nach einem der Ansprüche 10 bis 12, **dadurch gekennzeichnet, dass** beim Aufschmelzen des Materials des Befestigungsbereichs (6, 7) der Laserstrahl ortsfest ist und der Spritzenkörpers (1) so rotiert, dass eine ringförmige Aufheizzone entsteht.

15. Verfahren nach einem der Ansprüche 10 bis 12, **dadurch gekennzeichnet, dass** beim Aufschmelzen des Materials des Befestigungsbereichs (6, 7) der Spritzenkörper (1) ortsfest ist und der Laserstrahl so bewegt wird, dass eine ringförmige Aufheizzone entsteht.

## Claims

1. A syringe body (1) which comprises an at least partly cylindrical portion (2), wherein a cannula (4) is fastened in an axial end region (3) of the cylindrical portion (2), wherein the cannula (4) is arranged in a receiving channel (5) in the axial end region (3) of the cylindrical portion (2), and wherein the cannula (4) is fixed in the receiving channel (5) at least at one fastening region (6, 7) by means of material of the cylindrical portion (2), which, once melted, lies in a sealed manner around the circumference of the cannula (4) and forms a connection between the cylindrical portion (2) and the cannula (4),
**characterised in that**
at least two fastening regions (6, 7) arranged at the axial distance (a) are provided and fix the cannula (4) in the axial end region (3) of the cylindrical portion (2), wherein a region is arranged between the two fastening regions (6, 7) and in this region there is no connection between the cylindrical portion (2) and cannula (4) by means of molten material of the cylindrical portion (2).

2. The syringe body according to Claim 1, **characterised in that** two fastening regions (6, 7) are provided, of which one is arranged at the end of the axial end region (3) of the cylindrical portion (2).

3. The syringe body according to Claim 2, **characterised in that** the fastening region (6) arranged at the end of the axial end region (3) of the cylindrical portion (2) forms a tapered area (8) or a convex area on the outer circumference.

4. The syringe body according to one of Claims 1 to 3, **characterised in that** two fastening regions (6, 7) are provided, of which one is arranged at the axial end of the cannula (4) facing the interior (9) of the cylindrical portion (2).

5. The syringe body according to Claim 4, **characterised in that** the interior (9) of the syringe body (1) widens conically (10) from the fastening region (7) at the axial end of the cannula (4), wherein the surface (11) of the fastening region (7) has a planar or a curved, in particular convex, form.

6. The syringe body according to Claim 5, **characterised in that** the surface (11) of the fastening region (7) and the axial end of the cannula (4) terminate flushly.

7. The syringe body according to one of Claims 1 to 6, **characterised in that** the cylindrical portion (2) consists of glass.

8. A method for producing a syringe body (1), in which a cannula (4) is inserted into a receiving channel (5) in the axial end region (3) of an at least partly cylindrical portion (2) of the syringe body (1) and material of the axial end region (3) of the cylindrical portion (2) is melted in at least one fastening region (6, 7) by a heat source, in that the material is then left to cool, such that it lies in a sealed manner around the cannula (4) and forms a connection between the axial end region (3) of the cylindrical portion (2) and the cannula (4),
**characterised in that**
the melting and cooling process is performed in at least two fastening regions (6, 7) of the axial end region (3) of the cylindrical portion (2), said fastening portions being arranged at the axial distance (a), wherein a region is left between the two fastening regions (6, 7) and in this region a melting and cooling process is not performed, such that a connection between the cylindrical portion (2) and cannula (4) by means of molten material of the cylindrical portion (2) is not produced in this region.

9. The method according to Claim 8, **characterised in that** the heat source emits an electromagnetic radiation.

10. The method according to Claim 8 or 9, **characterised in that** the melting is carried out by means of a laser, in particular by means of an Nd:YAG laser or a diode laser or a CO₂ laser.

11. The method according to one of Claims 8 to 10, **characterised in that** the material is melted and cooled sequentially in the at least two fastening regions (6, 7).

12. The method according to one of Claims 8 to 10, **characterised in that** the material is melted and cooled simultaneously in the at least two fastening regions (6, 7).

13. The method according to one of Claims 10 to 12, **characterised in that** the laser light is directed onto the fastening region (6, 7) by means of a focussing optics (12) such that an annular heating zone is formed there.

14. The method according to one of Claims 10 to 12, **characterised in that**, when the material of the fastening region (6, 7) melts, the laser beam is stationary and the syringe body (1) rotates such that an annular heating zone is created.

15. The method according to one of Claims 10 to 12, **characterised in that**, when the material of the fastening region (6, 7) melts, the syringe body (1) is stationary and the laser beam is moved such that an annular heating zone is created.

## Revendications

1. Corps de seringue (1) comportant une section au moins partiellement cylindrique (2), dans lequel une canule (4) est fixée dans la région d'extrémité axiale (3) de la section cylindrique (2), dans lequel la canule (4) est agencée dans un canal d'admission (5) dans la région d'extrémité axiale (3) de la section cylindrique (2) et dans lequel la fixation de la canule (4) dans le canal d'admission (5) est réalisée dans au moins une région de fixation (6, 7), par du matériau de la section cylindrique (2) s'appliquant de façon étanche sur le pourtour de la canule (4) suite à une fusion et formant une liaison entre la section cylindrique (2) et la canule (4),
**caractérisé en ce que**
il est prévu au moins deux régions de fixation (6, 7) agencées avec un espacement axial (a), lesquelles fixent la canule (4) dans la région d'extrémité axiale (3) de la section cylindrique (2), dans lequel une région dans laquelle aucune liaison par fusion de matériau de la section cylindrique (2) n'existe entre la section cylindrique (2) et la canule (4) est agencée entre les deux régions de fixation (6, 7).

2. Corps de seringue selon la revendication 1, **caractérisé en ce qu'**il est prévu deux régions de fixation (6, 7), dont l'une est agencée à l'extrémité de la région d'extrémité axiale (3) de la section cylindrique (2).

3. Corps de seringue selon la revendication 2, **caractérisé en ce que** la région de fixation (6) agencée à l'extrémité de la région d'extrémité axiale (3) de la section cylindrique (2) forme une surface conique (8) ou une surface convexe sur le pourtour extérieur.

4. Corps de seringue selon l'une des revendications 1 à 3, **caractérisé en ce qu'**il est prévu deux régions de fixation (6, 7), dont l'une est agencée à l'extrémité axiale de la canule (4) qui est tournée vers l'intérieur (9) de la section cylindrique (2).

5. Corps de seringue selon la revendication 4, **caractérisé en ce que** l'espace intérieur (9) du corps de seringue (1) s'élargit de façon conique (10) à partir de la région de fixation (7) à l'extrémité axiale de la canule (4), dans lequel la surface (11) de la région de fixation (7) présente une forme plane ou voutée, en particulier convexe.

6. Corps de seringue selon la revendication 5, **caractérisé en ce que** la surface (11) de la région de fixation (7) et l'extrémité axiale de la canule (4) se terminent en affleurement.

7. Corps de seringue selon l'une des revendications 1 à 6, **caractérisé en ce que** la section cylindrique (2) est constituée de verre.

8. Procédé pour la fabrication d'un corps de seringue (1), dans lequel une canule (4) est insérée dans un canal d'admission (5) dans la région d'extrémité axiale (3) d'une section au moins partiellement cylindrique (2) du corps de seringue (1), puis du matériau de la région d'extrémité axiale (3) de la section cylindrique (2) est fondu dans au moins une région de fixation (6, 7), à l'aide d'une source de chaleur, dans lequel le matériau est ensuite refroidi, de manière à s'appliquer de façon étanche autour de la canule (4) et à former une liaison entre la région d'extrémité axiale (3) de la section cylindrique (2) et la canule (4),
**caractérisé en ce que**
l'opération de fusion et de refroidissement est réalisée dans au moins deux régions de fixation (6, 7) de la région d'extrémité axiale (3) de la section cylindrique (2), lesquelles sont agencées avec un espacement axial (a), dans lequel il demeure une région entre les deux régions de fixation (6, 7) dans laquelle aucune opération de fusion et de refroidissement n'est réalisée, pour omettre la réalisation d'une liaison par fusion du matériau de la section cylindrique (2) entre la section cylindrique (2) et la canule (4) dans cette région.

9. Procédé selon la revendication 8, **caractérisé en ce que** la source de chaleur émet un rayonnement électromagnétique.

10. Procédé selon la revendication 8 ou 9, **caractérisé en ce que** la fusion est effectuée au moyen d'un laser, en particulier d'un laser Nd:YAG ou d'un laser à diodes ou d'un laser CO₂.

11. Procédé selon l'une des revendications 8 à 10, **caractérisé en ce que** la fusion et le refroidissement du matériau sont réalisés de façon séquentielle dans les au moins deux régions de fixation (6, 7).

12. Procédé selon l'une des revendications 8 à 10, **caractérisé en ce que** la fusion et le refroidissement du matériau sont réalisés simultanément dans les au moins deux régions de fixation (6, 7).

13. Procédé selon l'une des revendications 10 à 12, **caractérisé en ce que** la lumière laser est dirigée de telle façon vers la région de fixation (6, 7) au moyen d'une optique de focalisation (12), qu'il s'y forme une zone de chauffe en forme d'anneau circulaire.

14. Procédé selon l'une des revendications 10 à 12, **caractérisé en ce que** lors de la fusion du matériau de la région de fixation (6, 7), le rayon laser est immobile et le corps de seringue (1) effectue une rotation formant la zone de chauffe en forme d'anneau circulaire.

15. Procédé selon l'une des revendications 10 à 12, **caractérisé en ce que** lors de la fusion du matériau de la région de fixation (6, 7), le corps de seringue (1) est immobile et le rayon laser se déplace de manière à former une zone de chauffe annulaire.
